# EUROPÄISCHE PATENTSCHRIFT

(11) **EP 1 201 256 B1**
(45) Veröffentlichungstag und Bekanntmachung des Hinweises auf die Patenterteilung: **27.09.2006**
(21) Anmeldenummer: 01125747.4
(22) Anmeldetag: 29.10.2001
(51) Int. Cl.: A61L 31/06, A61L 31/14, A61B 17/11

(54) **Bioresorbierbare Nervenleitschiene**
Bioresorbable nerve guidance channels
Canaux de guidage biorésorbables pour les nerfs

(30) Priorität: 28.10.2000 DE 10053611
(43) Veröffentlichungstag der Anmeldung: 02.05.2002
(73) Patentinhaber: DEUTSCHE INSTITUTE FÜR TEXTIL- UND FASERFORSCHUNG STUTTGART Stiftung des öffentlichen Rechts, 73770 Denkendorf (DE)
(72) Erfinder: Müller, Erhard, 70565 Stuttgart (DE); Hierlemann, Helmut, 73033 Göppingen (DE); Planck, Heinrich, 72622 Nürtingen (DE); Schlosshauer, Burkhard, 72074 Tübingen (DE)
(74) Vertreter: Patentanwälte Ruff, Wilhelm, Beier, Dauster & Partner

(56) Entgegenhaltungen:
- WO-A-95/20359
- WO-A-99/11181
- DE-A- 3 323 430
- JP-A- 4 262 780
- JP-A- 4 322 657
- US-A- 5 656 605
- US-A- 6 090 117
- STEUER H ET AL: "Biohybride nerve guide for regeneration: degradable polylactide fibers coated with rat Schwann cells." NEUROSCIENCE LETTERS. IRELAND 31 DEC 1999, Bd. 277, Nr. 3, 31. Dezember 1999 (1999-12-31), Seiten 165-168, XP002222872 ISSN: 0304-3940

## Beschreibung

Die Erfindung betrifft eine bioresorbierbare Nervenleitschiene mit einem mikroporösen Leitrohr aus Polymeren von Hydroxycarbonsäuren, wobei die Porosität einen Stoffwechsel durch die Rohrwandung erlaubt, aber den Durchgang von Zellen verhindert, und gegebenenfalls mehreren in dem Leitrohr angeordneten Monofilamenten aus Polymeren von Hydroxycarbonsäuren.

Bei einer Schädigung von Nervenleitungen im zentralen oder peripheren Nervensystem, beispielsweise durch Verletzungen, sind die Nervenzellen zwar in der Lage, neue Axone wachsen zu lassen, diese finden jedoch in der Regel nicht oder nur durch Zufall das andere Nervenende. Deshalb ist man dazu übergegangen, zur Überbrückung der Fehlstelle der Nervenleitung sogenannte Nervenleitschienen anzuordnen, die dem Axon eine gerichtete Orientierungshilfe für das Wachstum gibt.

Steuer et al. beschreiben in Neuroscience Letters 277 (1999) 165-168 biohybride Nervenleiteinrichtungen, in denen abbaubare Polylactidfasern mit Schwannschen Zellen überzogen sind.

DE 33 23 430 A offenbart ein Implantat mit einem Kanal und darin angeordneten längsgerichteten monofilen Fäden zur Überbrückung von Defekten an Rückenmark und peripheren Nerven.

WO 99/11181 A beansprucht einen Nervenleitkanal mit mehreren Hohlleitungen (Lumen) aus bioverträglichem Polymermaterial.

WO 95/20359 betrifft eine langgestreckte Struktur zur Förderung des Wachstums von Nerven, die in einer Umhüllung therapeutische Mittel zur Nervenwachstumsstimulation und Führungsfilamente enthält.

US 5,656,605 beschreibt ein Rohr mit dünnen Führungsfilamenten, die keine Profilierung aufweisen.

US 6,090,117 betrifft ein Rohr, das rohrförmige gewebt ist und dicht oder porös sein kann. Hier wird die Resorbierbarkeit durch Vernetzung in Kombination mit Kollagen beeinflusst. Das Kollagen ist jedoch ein biologisches und potentiell kontagiöses Material, das physiologisch unterwünscht ist.

JP 04-322657 und JP 04-262780 schlagen beide flache Platten mit parallelen Rillen vor, die durch Lithographieverfahren ausgebildet werden, und nicht porös sind.

Es ist auch bereits bekannt, solche Nervenleitschienen aus biologisch abbaubarem Material, insbesondere aus Polymeren von Hydroxycarbonsäuren auszubilden. Dadurch wird erreicht, dass die Nervenleitschiene sich dann, wenn die Nervenbahn wieder regeneriert ist, von selbst auflöst, so dass eine zweite Operation, die sonst für die Entfernung der Nervenleitschiene erforderlich wäre, entfällt.

Da in einem Nerv normalerweise mehrere parallele Leitungen angeordnet sind, wurde auch bereits vorgeschlagen, in einem biologisch abbaubaren Leitrohr Monofilamente in Form von Mikrohohlfasern anzuordnen. Die Ergebnisse waren jedoch bisher nicht zufriedenstellend.

Der Erfindung liegt daher die Aufgabe zugrunde, eine Nervenleitschiene zu schaffen, die eine Beschleunigung eines gerichteten Wachstums von funktionsfähigen Nervenzellen erlaubt.

Diese Aufgabe wird dadurch gelöst, dass das Leitrohr in Form einer rohrförmigen Membran vorliegt, die Monofilamente massiv ausgebildet sind, die Filamente einen Durchmesser von 30 bis 200 Mikrometern aufweisen, die Innenfläche des Rohres und/oder die Oberfläche der Monofilamente eine Orientierungshilfe zur längsorientierten Besiedelung mit Schwannschen Zellen aufweisen, und dadurch, dass als Orientierungshilfe eine Längsprofilierung vorgesehen ist.

Schwannsche Zellen bzw. deren Vorläuferzellen begünstigen das Wachstum von Axonen durch Nervenleitschienen und bilden später eine Umhüllung bzw. Scheide um die gewachsenen Axone. Schwannsche Zellen wurden bereits in Nervenleitschienen gegeben, um das Wachstum der Axone zu fördern. Der Erfindung liegt der Gedanke zugrunde, die Schwannschen Zellen in Längsorientierung aneinandergereiht entlang des Leitrohrs und/oder entlang der Monofilamente wachsen zu lassen, wodurch eine zwangsweise Längsorientierung der Axone erreicht wird, so dass durch das geradlinige Wachstum der Axone eine schnellere Verbindung der Nervenenden erreicht wird.

Dadurch, dass die Orientierungshilfe an der Innenseite des Leitrohrs bzw. an der äußeren Oberfläche der Monofilamente vorgesehen ist, stehen die Schwannschen Zellen bei ihrem Wachstum und später auch die Axone in Verbindung mit dem Innenraum des Leitrohres, der durch die Porosität des Leitrohres mit für den Stoffwechsel erforderlichen Substanzen versorgt werden kann.

Die Porengrösse der porösen Wandung bzw. Membran des Leitrohres liegt im Bereich von 0,1 bis 50 µm, vorzugsweise von 0,5 bis 3 µm. Bei dieser Porengrösse können Nährmedien und darin enthaltener Sauerstoff durch die Wandung des Leitrohres gelangen. Das störende Einwachsen von Bindegewebszellen, die ausserhalb der Nervenleitschiene vorhanden sind, wird jedoch verhindert.

Der Rohrinnendurchmesser liegt vorzugsweise im Bereich von 0,5 bis 10 mm, insbesondere im Bereich von 1 bis 5 mm. Dies entspricht in etwa der Dicke der in der Natur vorkommenden Nerven.

Die Herstellung des Leitrohres mit der porösen Wandung kann anhand bekannter Membrantechniken vorgenommen werden. Eine Möglichkeit ist die Phasenumkehr- bzw. Phaseninversionstechnik. Hierzu kann eine Lösung des biologisch abbaubaren Polymers in ein Bad bereits in Rohrform extrudiert werden, wobei das Bad mit dem Lösungsmittel für das Polymer mischbar ist, aber kein Lösungsmittel für das Polymer selbst ist. Eine weitere geeignete Membrantechnik ist die Gefriertrocknung. Hierzu kann ein Stab geeigneten Durchmessers und geeigneter Form mit einer Lösung des Polymers beschichtet und diese dann durch Gefriertrocknung in die feste Form überführt werden, wobei sich die Poren beim Trocknen ausbilden.

Die Polymere können Homopolymere, Copolymere und Terpolymere der Hydroxycarbonsäuren, Carbonate oder Lactone sein, wobei Copolymere und Terpolymere bevorzugt sind. Geeignete Monomere sind Glycolid, Lactid, insbesondere in der L- oder DL-Form, Trimethylcarbonat (TMC), Dioxanon, β-Hydroxybuttersäure und epsilon-Caprolacton. Als Beispiele für geeignete Polymermaterialien sind Polyglycolid, Polylactid, Polycaprolacton, Polytrimethylencarbonat, Polydioxanon, Polyhydroxybuttersäure sowie Copolymere, Terpolymere oder Blends dieser Polymere zu nennen.

Durch geeignete Auswahl der Monomeren und durch entsprechend gesteuerte Mengenverhältnisse kann die Resorbierbarkeitsdauer bzw. deren Halbwertszeit eingestellt werden. Dies gilt sowohl für das Leitrohr als auch für die Monofilamente. In der Regel soll innerhalb von sechs Monaten die Leitschiene verschwunden oder soweit aufgelöst sein, dass ein normaler Stoffwechsel möglich ist.

Schwannsche Zellen haben die Eigenschaft, sich als Monolayer an Oberflächen anzusiedeln und auf diesen zu wachsen. Erfindungsgemäss ist es deshalb mit Vorteil vorgesehen, die Fläche, an der sich die Schwannschen Zellen anlagern, in Längsrichtung in schmale Leitflächen zu unterteilen, entlang derer sich die Schwannschen Zellen in Längsrichtung lanzettenförmig anlagern können. Hierzu sind die Innenfläche des Leitrohres und/oder die Oberfläche der Monofilamente mit Vorteil mit Längsrippen und dazwischenliegenden Längsnuten versehen, wobei sowohl die Längsrippen als auch die Längsnuten als schmale axiale Leitflächen für die Schwannschen Zellen dienen können. Die Breite der Rippen und/oder Nuten liegt vorzugsweise in der Grössenordnung der Breite einer lanzettenförmigen Schwannschen Zelle, wodurch eine längsgerichtete Aneinanderreihung der Schwannschen Zellen in Form einer Kette erreicht wird. Die Übergänge zwischen den Längsrippen und den dazwischenliegenden Tälern bzw. Längsnuten sind vorzugsweise winkelförmig als Kanten ausgebildet. Die Axone können dann später nach dem Implantieren der Leitschiene entlang der Ketten der Schwannschen Zellen in gerader Linie wachsen. Die Breite der Längsrippen und vorzugsweise auch der Nuten liegt vorzugsweise im Bereich von 5 bis 30 µm. Die Tiefe der Nuten ist vorzugsweise nicht grösser als 10 µm und liegt insbesondere im Bereich von 5 bis 10 µm.

Die Innenfläche des Leitrohres und/oder die Oberfläche der Monofilamente können mit Vorteil mit einer Anwachshilfe zum schnelleren Besiedeln von Schwannschen Zellen versehen sein. Hierzu eignen sich insbesondere Beschichtungen mit Peptiden bzw. Polypeptiden, wobei insbesondere Polylysin bevorzugt ist. Die für eine Beschichtung mit biologisch aktiven Molekülen einzusetzenden Polyamine oder Polypeptide können beispielsweise aus extracellulären Matrixproteinen oder Enzymen abgeleitet sein. Es reicht aus, nur wenige Schwannsche Zellen bzw. Precursorzellen von Schwannschen Zellen in die Nervenleitschiene einzubringen. Diese vermehren sich dann in der gewünschten Weise entlang der Leitschiene. Weiterhin vorteilhaft ist es, die Innenfläche des Leitrohres und/oder die Oberfläche der Monofilamente zu hydrophilisieren. Dies kann in geeigneter Weise durch eine Plasmabehandlung in Gegenwart von Sauerstoff vorgenommen werden. Dadurch wird eine bessere Haftung der Anwachshilfe, insbesondere der Peptide erreicht.

Weiterhin ist es möglich und bevorzugt, auch an der Aussenseite der Nervenleitschiene entsprechende Anwachshilfen für Bindegewebszellen, insbesondere Fibroblasten vorzusehen, um das Wachsen von Bindegewebe um die Nervenleitschiene zu fördern, in das der Nerv dann nach Resorption der Leitschiene eingebettet liegt.

Bei einer besonders bevorzugten Ausführungsform der Erfindung, die auch unabhängig von der Orientierungshilfe für die Schwannschen Zellen vorgesehen sein kann, ist die Nervenleitschiene so aufgebaut, dass die Resorbierbarkeit der Leitschiene über ihre Länge abnimmt. Versuche haben gezeigt, dass es vorteilhaft ist, wenn der Nerv an den Stellen, an denen er bereits nachgewachsen ist und an denen bereits eine Ummantelung des Axons mit den Schwannschen Zellen erfolgt ist, möglichst bald freigelegt wird, um einen normalen Stoffwechsel mit der Umgebung zu ermöglichen. Zu diesem Zeitpunkt besteht die Gefahr einer Fehlorientierung des Axons nicht mehr und auch äussere Zellen können das Wachstum nicht mehr hemmen. Da das Wachstum der Axone vom proximalen Nervenende aus erfolgt, ist erfindungsgemäss vorgesehen, dass die Nervenleitschiene am proximalen Ende schneller resorbierbar ist als am distalen Ende. Da der hydrolytische Abbau der Polymeren der Hydroxycarbonsäuren bereits kurz nach dem Implantieren der Nervenleitschiene beginnt, wird die unterschiedliche Resorptionsdauer der Nervenleitschiene über ihre Länge mit Vorteil durch voneinander unterschiedliche Polymere erreicht. Diese kann über die unterschiedliche Zusammensetzung, das heisst über die Verwendung unterschiedlicher Monomere oder Monomerverhältnisse sowie auch durch unterschiedliches Molekulargewicht erzielt werden.

Die Resorptionsdauer kann kontinuierlich oder auch diskontinuierlich vom proximalen zum distalen Ende zunehmen. Eine kontinuierliche Zunahme kann insbesondere dadurch erreicht werden, dass die vorgebildeten Leitrohre und/oder Monofilamente in Abhängigkeit von ihrer Länge unterschiedlich stark mit Gammastrahlen behandelt werden. Dies kann durch unterschiedliche Verweildauer erreicht werden. Bei einer bevorzugten Herstellungsweise können die vorgebildeten Teile der Leitschiene in Bleikammern angeordnet werden, deren Wandstärke von einem Ende zum anderen Ende zunimmt, wodurch erreicht wird, dass die Bestrahlungsintensität entsprechend dem Anstieg der Wandstärke abnimmt.

Eine Degradationsdauer von 0,5 bis 6 Monaten entlang der Länge der Leitschiene ist bevorzugt. Die Länge der Leitschiene hängt von der Grösse der zu überbrückenden Strecke ab und liegt im Normalfall zwischen 1 und 10 cm.

Eine unterschiedliche Degradationsdauer über die Länge der Leitschiene ist aber auch durch Verwendung verschiedener Polymere möglich. Es ist bekannt, dass Lactid enthaltende Polymere eine längere Degradationsdauer besitzen als solche, die Glycolid enthalten. Durch den entsprechenden Copolymeranteil bzw. Terpolymeranteil können die Degradationsdauern gesteuert werden. Beispielsweise liegt die Degradationsdauer von epsilon-Caprolacton-Lactid-Copolymer (50/50) bei etwa einem Monat, und bei einem entsprechenden Copolymer mit einem Monomerverhältnis von 90/10 bei drei Monaten. Bei einem epsilon-Caprolacton-Trimethylcarbonat-Glycolid-Polymer liegt die Degradationsdauer unter einem Monat.

Die Wandstärke der Wand bzw. Membran des Leitrohrs liegt mit Vorteil im Bereich von 50 bis 400 µm. Es ist bevorzugt, die Wandstärke über die Länge gesehen im wesentlichen konstant zu halten, um die Stoffwechselvorgänge durch die poröse Wandung hindurch nicht durch zu dicke Wandungen zu beeinträchtigen. Trotzdem ist es mit Vorteil möglich, die unterschiedliche Degradationsdauer auch durch einen unterschiedlichen Schichtaufbau zu steuern. So kann das Leitrohr auch aus mehreren in ihrer Länge abgestuften Schichten gebildet sein, wobei die untere, längste Schicht aus leicht resorbierbarem Material besteht und die nachfolgenden Schichten, die entsprechend abgestuft kürzer sind, in der Degradationsdauer zunehmen. An den mehrfach beschichteten Stellen ist die unterste schnellresorbierbare Schicht dann durch weniger resorbierbare Deckschichten geschützt, so dass auf diese Weise eine zeitlich gesteuerte über die Länge zunehmende Resorptionsdauer erreicht wird. Es sind auch Kombinationen von unterschiedlicher Zusammensetzung und Bestrahlung möglich.

Die Ausbildung der Monofilamente als massive Kompaktfasern verleiht ihnen die notwendige Stabilität und erleichtert auch die Ausbildung der längsstrukturierten Oberfläche als Orientierungshilfe für die Schwannschen Zellen.

Die Herstellung der Monofilamente erfolgt mit besonderem Vorteil durch Extrusion durch entsprechend geformte Düsen mit in etwa mäanderförmiger Umfangslinie. Die Einstellung der zunehmenden Resorptionsdauer von proximalem Ende zu distalem Ende wird mit Vorteil durch die oben erwähnte Bestrahlung vorgenommen. Die Monofilamente haben einen Durchmesser, der vorzugsweise im Bereich von 30 bis 200 µm, insbesondere 100 bis 150 µm liegt.

Im Leitrohr können viele Monofilamente angeordnet sein. In der Regel sind es 10 bis 1000, je nach Grösse des Leitrohrs. Der Rohrinnenquerschnitt wird jedoch nicht vollständig mit Monofilamenten ausgefüllt, da die Zellen einerseits Platz zum Wachstum benötigen, andererseits auch Platz für Nährmedium im Rohr innen benötigt wird. Normalerweise ist der Innenquerschnitt des Leitrohres zu etwa ein Drittel bis ein Halb mit Monofilamenten gefüllt. Mit besonderem Vorteil sind sowohl die Innenfläche des Leitrohres als auch die Oberflächen der Monofilamente zur Besiedelung mit Schwannschen Zellen bestimmt und mit den entsprechenden Orientierungshilfen für diese ausgerüstet.

In die Leitschiene, insbesondere in deren resorbierbares Material können Wirkstoffe und/oder Wachstumsfaktoren eingelagert sein, die spätestens beim biologischen Abbau des resorbierbaren Materials freigesetzt werden. So können in die Leitschiene mit Vorteil säurebindende Puffersubstanzen eingelagert sein. Bei der Hydrolyse der Hydroxycarbonsäurepolymere entstehen Bruchstücke oder Monomere, die Carboxylgruppen aufweisen. Die dadurch mögliche unerwünschte Senkung des pH-Wertes kann durch die Puffer abgefangen werden. Solche Puffer sind vorzugsweise in den resorbierbaren Polymeren vorhanden.

Weiterhin können Antibiotika eingelagert sein, die insbesondere durch retardierte Freigabe, Infektionen nach der Implantation der Leitschiene verhindern.

Leitschienen, die zum Verbinden mit dem Umgebungsgewebe vorgesehen sind, besitzen mit besonderem Vorteil an Enden, die mit dem Umgebungsgewebe verbunden werden sollen, Hemmstoffe für Stoppsignale des Umgebungsgewebes. Diese Stoppsignale verhindern normalerweise das Wachsen von Axonen und die Verbindung mit freigelegten Nervenenden des Rückenmarks. Diese Stoppsignale können durch Hemmstoffe wie Antikörper oder Enzym-Hemmstoffe an ihrer Stoppfunktion gehemmt werden.

Der von den Monofilamenten und der Anfangsbesiedelung mit Schwannschen Zellen nicht eingenommene Innenraum der Leitrohre ist vorzugsweise mit einem Nährgel für Schwannsche Zellen gefüllt. Dieses liegt vorzugsweise als wässriges Gel vor, in das mit besonderem Vorteil Wachstumsfaktoren für die Schwannschen Zellen eingelagert sein können.

Die erfindungsgemässen Nervenleitschienen sind vorzugsweise flexibel ausgebildet, was durch entsprechende Auswahl der Polymere auch ohne Zugabe von Weichmachern möglich ist. Falls erwünscht können die Nervenleitschienen auch Verzweigungen besitzen. Rohrförmige Verzweigungen können beispielsweise hergestellt werden, indem zur Formgebung zerlegbare Y-förmige Stäbe beschichtet werden, wie dies bei Gefässprothesen bekannt ist.

Die Herstellung der zur Implantation vorbereiteten Leitschienen erfolgt vorzugsweise, indem das Leitrohr und die Monofilamente getrennt voneinander vorbereitet werden und die Monofilamente in die Leitrohre eingeschoben werden. Dabei sind die Monofilamente vorzugsweise vor dem Einschieben mindestens teilweise mit Schwannschen Zellen bzw. Vorläuferzellen besiedelt.

Gegenstand der Erfindung sind demgemäss auch die Monofilamente für sich allein ausgerüstet mit der Orientierungshilfe, insbesondere der Längsprofilierung, und gegebenenfalls der Anwachshilfe für die Schwannschen Zellen, insbesondere mit der mindestens teilweisen Besiedelung mit diesen Zellen bzw. ihren Vorläufern.

Weitere Merkmale der Erfindung ergeben sich aus der nachfolgenden Beschreibung einer bevorzugten Ausführungsform der Erfindung in Verbindung mit der Zeichnung und den Ansprüchen.

In der Zeichnung zeigen:
Figur 1 eine perspektivische Ansicht eines Längsabschnittes einer erfindungsgemässen Nervenleitschiene,
Figur 2 einen Teilquerschnitt durch die poröse Membranwand der Leitschiene nach Figur 1,
Figur 3 eine perspektivische Teilansicht eines Monofils für die Leitschiene nach Figur 1, und
Figur 4 in symbolisierter Form eine den Innenraum des Leitrohres ausfüllende Gelmatrix.

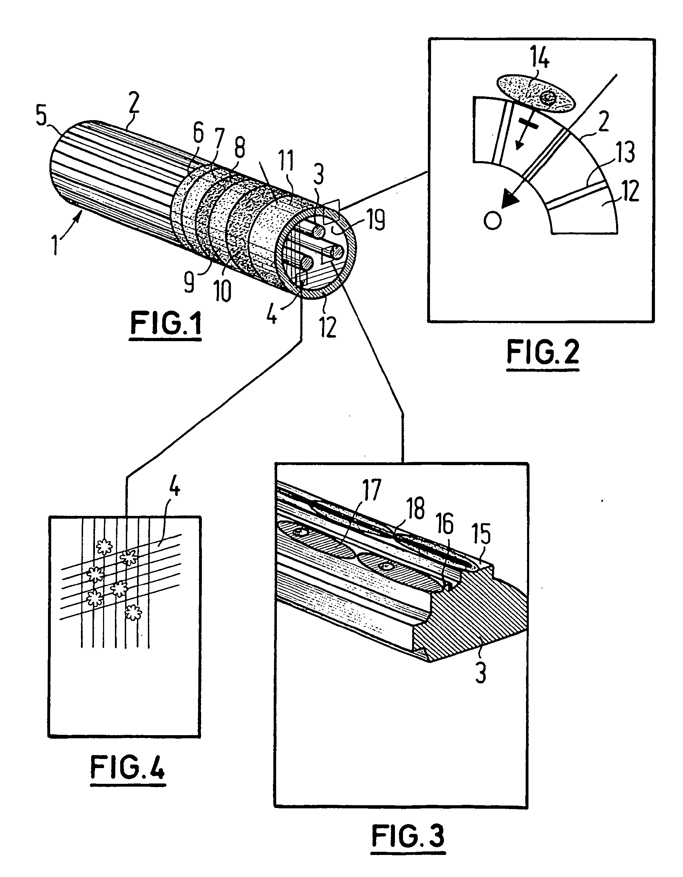

Bei der in der Zeichnung dargestellten Ausführungsform weist eine Leitschiene 1 ein Leitrohr 2 auf, in dessen Innenraum ca. 10 bis 50 Monofilamente 3 (in der Zeichnung nur drei in vergrössertem Massstab dargestellt) in Längsrichtung angeordnet sind. Die Monofilamente 3 sind in ein Gel 4 (Figur 4) eingebettet, das sie in einem Abstand voneinander hält.

Das Leitrohr 2 besteht aus bioresorbierbaren Polymeren von Hydroxycarbonsäuren und besitzt einen Aufbau in Form von mehreren unterschiedlich langen Schichten 5 bis 11. Diese unterscheiden sich auch in ihrer Zusammensetzung, die so abgestimmt ist, dass die innerste Schicht 5 am proximalen Ende am schnellsten degradierbar ist, nämlich innerhalb von 0,5 Monaten, wogegen die äusserste und kürzeste Schicht 11 erst innerhalb von 6 Monaten degradiert wird. Die Degradierdauer der Schichten 6 bis 10, die abgestuft verkürzt sind, liegt entsprechend abgestuft ansteigend dazwischen.

Dieser Schichtaufbau kann durch abgestuftes Tauchen eines entsprechend vorgebildeten Stabes, insbesondere aus PTFE (Polytetrafluorethylen), in Polymerlösungen der verschiedenen Polymere erreicht werden, wobei der Stab für Schicht 5 am tiefsten und für die Schichten 6 bis 11 immer weniger tief eingetaucht wird.

Eine Porosität der als semipermeable Membran ausgebildeten Rohrwandung 12 wird durch Gefriertrocknung der Polymerlösungen nach dem Tauchen erreicht.

Der in Figur 3 dargestellte Teilquerschnitt zeigt Poren 13, die einen Austausch von Nährmedium und Sauerstoff erlauben, aber das Einwachsen von Zellen wie Fibroblasten 14, verhindern.

Die Monofilamente 3 sind kompakt, das heisst massiv ausgebildet und besitzen ein Längsprofil aus Längsrippen 15 und dazwischenliegenden Längsnuten 16, die jeweils in etwa die gleiche Breite haben und über den gesamten Aussenumfang der Monofilamente vorhanden sind. Die Monofilamente bestehen aus einem Polymeren von Hydroxycarbonsäuren, das eine Resorptionsdauer von ca. sechs Monaten in vivo besitzt. Sie werden durch Extrusion aus einer entsprechend geformten Düse hergestellt.

Durch eine abgestufte Behandlung mit Gammastrahlen ist die Resorptionsdauer im wesentlichen kontinuierlich abnehmend eingestellt und beträgt am proximalen Ende wie die Schicht 5 des Leitrohres 2 nur noch 0,5 Monate.

Die Oberfläche der Monofilamente ist mit Polylysin beschichtet (nicht dargestellt), das die Besiedelung mit und das Wachstum von Schwannschen Zellen 17 bzw. deren Vorläuferzellen begünstigt. Diese Zellen lagern sich auf den Längsrippen 15 und/oder in den Längsnuten 16 in lanzettenförmiger Längsausrichtung hintereinander an und begünstigen so nach Implantation das regenerierende Einwachsen eines Axons 18 einer Nervenzelle vom proximalen Nervenende aus entlang der Kette der Schwannschen Zellen, wie dies in Figur 3 dargestellt ist. Die Haftung der Polylysinschicht kann durch vorhergehende Plasmabehandlung der Monofilamente in Gegenwart von Sauerstoff begünstigt werden, wodurch eine Hydrophilisierung der Polymeroberfläche erreicht wird.

In gleicher Weise ist die Innenfläche 19 des Leitrohres 2 mit Längsrippen und Längsnuten versehen und mit Polylysin beschichtet. Auch dort werden in gleicher Weise Schwannsche Zellen bzw. deren Vorläuferzellen ausgerichtet. Auf diese Weise wird erreicht, dass das Nervenwachstum zwangsläufig entlang der Leitschienen in einer Vielzahl von parallelen, aber voneinander unabhängigen Leitungen erfolgt.

Die Ausbildung der Längsrippen und Längsnuten an der Innenfläche 19 des Leitrohres 2 kann dadurch erreicht werden, dass ein entsprechender Stab, auf dem das Leitrohr abgeformt wird, eine entsprechend strukturierte Oberfläche besitzt.

In das Gel 4 im Inneren des Leitrohres sind die Zellproliferation der Schwannschen Zellen begünstigende Wachstumsfaktoren eingelagert sowie gegebenenfalls Nährstoffe für diese Zellen. Die Schwannschen Zellen ihrerseits geben Faktoren ab, die das Axon-Wachstum aktivieren und diese Axone veranlassen, entlang der längs ausgerichteten Schwannschen Zellen zu wachsen. Da das Axon-Wachstum vom proximalen Nervenende aus beginnt und dort die Heilung am schnellsten abgeschlossen ist, wird die Stützkonstruktion der Nervenleitschiene an diesem Ende, zeitlich gesehen, zuerst nicht mehr benötigt. Deshalb kann diese Stelle durch Degradation, insbesondere hydrolytische Degradation, abgebaut werden, nachdem sich die Schwannschen Zellen als Mantel um die nachgewachsenen Axone gelegt haben. Mit fortschreitendem Wachstum der Axone verliert die Nervenleitschiene ihre Funktion und kann fortschreitend und schliesslich vollständig entfallen, was durch die progressive Resorptionsdauer erreicht wird.

An der Aussenfläche der Leitschiene 1 bildet sich eine Hülle aus Fibroblasten 14, die die schützende Funktion des Leitrohres übernehmen. Das Anwachsen solcher Zellen kann ähnlich wie bei den Monofilamenten durch hydrophilisierende Plasmabehandlung in Gegenwart von Sauerstoff und/oder durch Peptidbeschichtung begünstigt werden.

Die Leitrohre 2 können vor dem Einschieben der Monofilamente 3 mit dem Gel 4 befüllt sein, beispielsweise einem Fibrin- oder Collagengel. Durch das Einschieben der Monofilamente wird dann überschüssiges Gel verdrängt. Es ist aber auch möglich, das Gel zusammen mit den Monofilamenten oder nach dem Einbringen dieser einzupressen. Vorzugsweise ist eine Besiedelung mit Schwannschen Zellen schon vor dem Einbringen der Monofilamente in das Leitrohr vorgenommen. Ein weiteres Wachstum findet dann nach dem Zusammenfügen statt.

Als Schwannsche Zellen bzw. deren Vorläufer werden vorzugsweise solche verwendet, die vom Patienten zuvor entnommen sind. Da nach Nervenverletzungen häufig eine mehrwöchige Wartezeit bis zur Resorption zerstörten Gewebes abgewartet werden muss, reicht die Zeit bis zur Implantation aus, um die erforderliche Menge an Schwannschen Zellen bzw. deren Vorläuferzellen zu züchten.

## Patentansprüche

1. Biologisch resorbierbare Nervenleitschiene (1) mit einem mikroporösen Leitrohr (2) aus Polymeren von Hydroxycarbonsäuren, wobei die Porosität einen Stoffwechsel durch die Rohrwandung (12) erlaubt, aber den Durchgang von Zellen (14) verhindert, und gegebenenfalls mehreren in dem Leitrohr (2) angeordneten Monofilamenten (3) aus Polymeren von Hydroxycarbonsäuren, **dadurch gekennzeichnet, dass** das Leitrohr in Form einer rohrförmigen Membran vorliegt, die Monofilamente(3) massiv ausgebildet sind, die Filamente einen Durchmesser von 30 bis 200 Mikrometern aufweisen, die Innenfläche (19) des Rohres (2) und/oder die Oberfläche der Monofilamente (3) eine Orientierungshilfe (15, 16) zur längsorientierten Besiedelung mit Schwannschen Zellen (17) aufweisen, und dadurch, dass als Orientierungshilfe eine Längsprofilierung vorgesehen ist.

2. Leitschiene nach Anspruch 1, **dadurch gekennzeichnet, dass** die Längsprofilierung von Längsrippen (15) und dazwischenliegenden Längsnuten (16) gebildet wird.

3. Leitschiene nach Anspruch 1 oder 2, **dadurch gekennzeichnet, dass** mindestens die Teile der Innenfläche (19) des Leitrohres (2) und/oder der Oberfläche der Monofilamente (3) eine Anwachshilfe für die Schwannschen Zellen aufweisen.

4. Leitschiene nach einem der vorhergehenden Ansprüche, **dadurch gekennzeichnet, dass** mindestens die mit Schwannschen Zellen zu besiedelnden Teile der Oberfläche (19) des Leitrohres (2) und/oder der Oberfläche der Monofilamente (3) hydrophilisiert sind, insbesondere durch eine Plasmabehandlung in Gegenwart von Sauerstoff.

5. Leitschiene nach einem der vorhergehenden Ansprüche, **dadurch gekennzeichnet, dass** mindestens die mit Schwannschen Zellen zu besiedelnden Teile der Innenfläche (19) des Leitrohres (2) und/oder der Oberfläche der Monofilamente (3) mindestens teilweise mit Schwannschen Zellen bzw. deren Vorläuferzellen besiedelt sind.

6. Biologisch resorbierbare Nervenleitschiene (1) nach dem Oberbegriff von Anspruch 1, insbesondere nach einem der vorhergehenden Ansprüche, **dadurch gekennzeichnet, dass** die Resorbierbarkeit der Leitschiene (1) über ihre Länge abnimmt und die Leitschiene (1) an einem proximalen Ende (5) schneller resorbierbar ist als an einem distalen Ende (11).

7. Leitschiene nach einem der vorhergehenden Ansprüche, **dadurch gekennzeichnet, dass** die Monofilamente (3) nur einen Teil des Innenquerschnitts des Leitrohres (2) ausfüllen und der verbleibende Teil vorzugsweise mit einem stimulierenden wässrigen Nährgel (4) für Schwannsche Zellen (17) ausgefüllt ist.

8. Leitschiene nach einem der vorhergehenden Ansprüche, **dadurch gekennzeichnet, dass** etwa ein Drittel bis ein Halb des Innenquerschnitts des Leitrohres (2) mit Monofilamenten (3) gefüllt ist.

9. Leitschiene nach einem der vorhergehenden Ansprüche, **dadurch gekennzeichnet, dass** in die Leitschiene (1), insbesondere in das resorbierbare Material von Leitrohr (2) und/oder Monofilamenten (3) Wirkstoffe und/oder Wachstumsfaktoren eingelagert sind, die spätestens beim biologischen Abbau des resorbierbaren Materials freigesetzt werden.

10. Leitschiene nach einem der vorhergehenden Ansprüche, **dadurch gekennzeichnet, dass** in die Leitschiene (1), insbesondere in das Innere des Leitrohres (2) säureabfangende Puffersubstanzen eingelagert sind.

11. Leitschiene nach einem der vorhergehenden Ansprüche, **dadurch gekennzeichnet, dass** in Enden der Leitschiene, die zum Verbinden mit dem Umgebungsgewebe vorgesehen sind, Hemmstoffe für Stoppsignale des Umgebungsgewebes eingelagert sind.

12. Monofilamente (3) aus biologisch resorbierbarem Material für Nervenleitschienen mit einer Orientierungshilfe (15, 16), insbesondere einer Längsprofilierung, zum längsorientierten Anwachsen von Schwannschen Zellen (17).

13. Monofilamente (3) aus biologisch resorbierbarem Material für Nervenleitschienen, **dadurch gekennzeichnet, dass** die Resorptionsdauer über die Länge der Monofilamente zunimmt.

14. Monofilamente nach Anspruch 12 oder 13, **dadurch gekennzeichnet, dass** mindestens ein Teil ihrer Oberfläche mit Schwannschen Zellen besiedelt ist.

## Claims

1. Bioresorbable nerve guidance channel (1) with a microporous guide tube (2) made of polymers of hydroxycarboxylic acids, the porosity permitting a metabolism through the tube wall (12) but preventing the passage of cells (14), and if appropriate a plurality of monofilaments (3) made of polymers of hydroxycarboxylic acids and arranged in the guide tube (2), **characterized in that** the guide tube is present in the form of a tubular membrane, the monofilaments (3) are of solid configuration, the filaments have a diameter of 30 to 200 micrometres, the inner surface (19) of the tube (2) and/or the surface of the monofilaments (3) having an orientation aid (15, 16) for longitudinally oriented colonization with Schwann cells (17), and **in that** a longitudinal profiling is provided as the orientation aid.

2. Guidance channel according to Claim 1, **characterized in that** the longitudinal profiling is made up of longitudinal ribs (15) and of longitudinal grooves (16) lying between these.

3. Guidance channel according to Claim 1 or 2, **characterized in that** at least parts of the inner surface (19) of the guide tube (2) and/or of the surface of the monofilaments (3) have a growth aid for the Schwann cells.

4. Guidance channel according to one of the preceding claims, **characterized in that** at least the parts of the surface (19) of the guide tube (2) and/or of the surface of the monofilaments (3) to be colonized with Schwann cells are hydrophilized, in particular by a plasma treatment in the presence of oxygen.

5. Guidance channel according to one of the preceding claims, **characterized in that** at least the parts of the inner surface (19) of the guide tube (2) and/or of the surface of the monofilaments (3) to be colonized with Schwann cells are at least partially colonized with Schwann cells or their precursor cells.

6. Bioresorbable nerve guidance channel (1) according to the preamble of Claim 1, in particular according to one of the preceding claims, **characterized in that** the resorbability of the guidance channel (1) decreases along its length, and the guidance channel (1) is more rapidly resorbable at a proximal end (5) than at a distal end (11).

7. Guidance channel according to one of the preceding claims, **characterized in that** the monofilaments (3) fill only part of the internal cross section of the guide tube (2), and the remaining part is preferably filled with a stimulating aqueous nutrient gel (4) for Schwann cells (17).

8. Guidance channel according to one of the preceding claims, **characterized in that** approximately a third to a half of the internal cross section of the guide tube (2) is filled with monofilaments (3).

9. Guidance channel according to one of the preceding claims, **characterized in that** active substances and/or growth factors are incorporated into the guidance channel (1), in particular into the resorbable material of guide tube (2) and/or monofilaments (3), said active substances and/or growth factors being released at the latest during the biodegradation of the resorbable material.

10. Guidance channel according to one of the preceding claims, **characterized in that** acid-absorbing buffer substances are incorporated into the guidance channel (1), in particular into the interior of the guide tube (2).

11. Guidance channel according to one of the preceding claims, **characterized in that** inhibitors for stop signals of the surrounding tissue are incorporated into ends of the guidance channel that are provided for connection to the surrounding tissue.

12. Monofilaments (3) made of bioresorbable material for nerve guidance channels with an orientation aid (15, 16), in particular a longitudinal profiling, for longitudinally oriented growth of Schwann cells (17).

13. Monofilaments (3) made of bioresorbable material for nerve guidance channels, **characterized in that** the resorption duration increases along the length of the monofilaments.

14. Monofilaments according to Claim 12 or 13, **characterized in that** at least part of their surface is colonized with Schwann cells.

## Revendications

1. Guide de nerf (1) biologiquement résorbable présentant un tube de guidage (2) microporeux en polymères d'acides hydroxycarboxyliques, la porosité admettant un métabolisme à travers la paroi (12) du tube, mais empêchant le passage de cellules (14), et le cas échéant plusieurs monofilaments (3) disposés dans le tube de guidage (2) en polymères d'acides hydroxycarboxyliques, **caractérisé en ce que** le tube de guidage se trouve sous forme d'une membrane en forme de tube, les monofilaments (3) présentent une réalisation massive, les filaments présentent un diamètre de 30 à 200 micromètres, la surface interne (19) du tube (2) et/ou la surface des monofilaments (3) présente un auxiliaire d'orientation (15, 16) pour la colonisation orientée longitudinalement par des cellules de Schwann (17) et **en ce qu'**on a prévu, comme auxiliaire d'orientation, un profil longitudinal.

2. Guide selon la revendication 1, **caractérisé en ce que** le profil longitudinal est formé par des nervures longitudinales (15) et des rainures longitudinales (16) situées entre celles-ci.

3. Guide selon la revendication 1 ou 2, **caractérisé en ce qu'**au moins les parties de la surface interne (19) du tube de guidage (2) et/ou de la surface des monofilaments (3) présentent un auxiliaire de croissance pour les cellules de Schwann.

4. Guide selon l'une quelconque des revendications précédentes, **caractérisé en ce qu'**au moins les parties à coloniser par les cellules de Schwann de la surface (19) du tube de guidage (2) et/ou de la surface des monofilaments (3) sont rendues hydrophiles, en particulier par un traitement au plasma en présence d'oxygène.

5. Guide selon l'une quelconque des revendications précédentes, **caractérisé en ce qu'**au moins les parties à coloniser par les cellules de Schwann de la surface interne (19) du tube de guidage (2) et/ou de la surface des monofilaments (3) sont au moins partiellement colonisées par des cellules de Schwann ou leurs précurseurs.

6. Guide de nerf (1) biologiquement résorbable selon le préambule de la revendication 1, en particulier selon l'une quelconque des revendications précédentes, **caractérisé en ce que** l'aptitude à la résorption du guide (1) diminue sur sa longueur et que le guide (1) est résorbable plus rapidement en une extrémité proximale (5) qu'en une extrémité distale (11).

7. Guide selon l'une quelconque des revendications précédentes, **caractérisé en ce que** les monofilaments (3) ne remplissent qu'une partie de la section interne du tube de guidage (2) et la partie résiduelle est de préférence remplie d'un gel nutritif (4) aqueux de stimulation pour les cellules de Schwann (17).

8. Guide selon l'une quelconque des revendications précédentes, **caractérisé en ce qu'**environ un tiers à la moitié de la section interne du tube de guidage (2) est rempli de monofilaments (3).

9. Guide selon l'une quelconque des revendications précédentes, **caractérisé en ce qu'**on a incorporé dans le guide (1), en particulier dans le matériau résorbable du tube de guidage (2) et/ou des monofilaments (3) des substances actives et/ou des facteurs de croissance qui sont libérés au plus tard lors de la dégradation biologique du matériau résorbable.

10. Guide selon l'une quelconque des revendications précédentes, **caractérisé en ce qu'**on a incorporé dans le guide (1), en particulier à l'intérieur du tube de guidage (2) des substances tampon piégeant les acides.

11. Guide selon l'une quelconque des revendications précédentes, **caractérisé en ce qu'**on a prévu dans les extrémités du guide, qui sont prévues pour la liaison avec le tissu environnant, des substances inhibitrices des signaux de blocage du tissu environnant.

12. Monofilaments (3) en matériau biologiquement dégradable pour guides de nerf avec un auxiliaire d'orientation (15, 16), en particulier un profil longitudinal pour la croissance orientée longitudinalement de cellules de Schwann (17).

13. Monofilaments (3) en matériau biologiquement résorbable pour guides de nerf, **caractérisés en ce que** la durée de résorption augmente sur la longueur des monofilaments.

14. Monofilaments selon la revendication 12 ou 13, **caractérisés en ce qu'**au moins une partie de leur surface est colonisée par des cellules de Schwann.
